Europäisches Patentamt

European Patent Office

Office européen des brevets

(11) Publication number: **0 294 098**
**A1**

# EUROPEAN PATENT APPLICATION

(21) Application number: 88304763.1

(51) Int. Cl.⁴: **C12Q 1/68**

(22) Date of filing: 26.05.88

(30) Priority: 29.05.87 US 55224
17.05.88 US 194982

(43) Date of publication of application:
07.12.88 Bulletin 88/49

(84) Designated Contracting States:
DE FR GB IT

(71) Applicant: **CITY OF HOPE**
**1500 East Duarte Road**
**Duarte California 91010-0269(US)**

(72) Inventor: **Wallace, Robert Bruce**
**1020 Buena Vista**
**South Pasadena California 91030(US)**

(74) Representative: **De Minvielle-Devaux, Ian**
**Benedict Peter et al**
**CARPMAELS & RANSFORD 43, Bloomsbury**
**Square**
**London WC1A 2RA(GB)**

(54) **Synthetic DNA probes.**

(57) A synthetic oligonucleotide hybridization probe comprising at least one sequence at least 12 nucleotides long, the probe forms duplexes in which at least 80% of the nucleotides are base paired with any nucleotide sequence that is either substantially of the form $(X_i...X_i)n$ in which X is any nucleotide; i which designates the number of nucleotides in the sequence is at least 2 and n is at least 2, or substantially of the form $[(X_i...X_i)mY]w$ in which X is any nucleotide, Y is any group of 1 or more nucleotides and is different from $(X_i...X_i)$, m is at least 1, w is 2, and i is at least 2, the number of nucleotides in said probe being less than 3i when i is greater than 3.

EP 0 294 098 A1

Xerox Copy Centre

# SYNTHETIC DNA PROBES

## Field of the Invention

This invention relates to synthetic oligonucleotides useful to identify intrinsic polymorphism of loci in human genomic DNA.

## Background of the Invention

The human genome contains several families of repetitive DNA sequences. Individual repetitive DNA families may be dispersed throughout the genome or may be organized in the form of long arrays of simple tandem repeats (STR). Some STR sequences have been referred to as minisatellites but have not been isolated as sequences of unique buoyant density. (Jeffreys, et al (1985) Nature 314:67; Jeffreys, et al. (1985) Nature 316:76 (1985); Jeffreys U.K. Patent Application GB 2 166 445 A published May 8, 1986.) Many of these minisatellites seem to be highly polymorphic due to variation in the number of copies of tandemly repeated units (VTNRs). Recently several loci which contain variable numbers of tandem repeats have been described. (Nakamura, et al., (1987) Science 235:1616-1632.)

Some human VTNR loci have been isolated as cloned DNA's and characterized. (Nakamura, et al., supra, n. 2; Wong, et al. (1986) Nucleic Acids Research 14:4605-4615.) Probes consisting of many tandem repeat units hybridize to multiple loci each having considerable restriction fragment length polymorphism (RFLP). The consequent individual specific hybridization pattern forms the basis of genetic fingerprinting.

## Summary of the Invention

This invention comprises synthetic oligonucleotide probes complementary to one or two repeat units of VTNR loci. Under high hybridization criteria, these probes hybridize locus specifically thus allowing the evaluation of the intrinsic polymorphism of individual loci.

## Detailed Description of the Invention

As the number of tandem repeat units of known minisatellite probes increases, specificity for DNA sequences of similar repeat unit length increases. (See, e.g., the references cited supra, note 1; Jeffreys, et al. (1986) Am. J. Hum. Genet. 39:11-24.)

Pursuant to this invention, probe length is limited to render hybridization dependent upon the degree of matching and the stringency of the hybridization criteria. As distinguished from the prior art, the probes of this invention provide greater specificity for a single locus or for a small number of loci included in a DNA sequence.

The synthetic oligonucleotide DNA hybridization probes of this invention comprise at least one sequence at least 12 nucleotides long which form duplexes in which at least about 80% of the nucleotides are base paired with any nucleotide sequence that is substantially of the form

(i) $(X_l...X_i)_n$ in which X is any nucleotide, i designates the number of nucleotides in the repeat and is at least 2, and n is at least 2, or substantially of the form

(ii) $[(X_l...X_i)mY]w$ in which X is any nucleotide, Y is any group of one or more nucleotides and is different from $(X_l...X_i)$, m is at least 1, w is 2 and i is at least 2, the number of nucleotides in said probe being less than 3i when i is greater than 3.

To accommodate either consensus or mixed genomic sequences, the probes of the invention may include either a single sequence or a mixture of sequences each at least 12 nucleotides long. The $(X_l...X_i)$ repeats may include minor sequence variations, hence the probes substantially conform to forms (i) and (ii).

A preferred embodiment comprises locus specific probes which hybridize at high criteria to form 100% matched duplexes. Interdependent constraints are thus placed both on the probe and on the locus.

Table I reports the sequences of 11 synthetic oligonucleotide probes (1, 2, and 4 to 12) which embody this invention, and one prior art probe (3).

TABLE I

SEQUENCES AND HYBRIDIZATION CHARACTERISTICS OF THE OLIGONUCLEOTIDE PROBES

| PROBE NO. | PROBE NAME | SEQUENCE 5'->3' | TOTAL LENGTH | LENGTH REPEAT UNIT | NO. REPEAT UNITS | CATEGORY OF HYBRIDIZATION TO HUMAN DNA |
|---|---|---|---|---|---|---|
| 1 | O-33.6-22 | $(CCTCCAGCCCT)_2$ | 22 | 11 | 2 | 1 |
| 2 | O-33.6-37 | GCCCTTCCTCCGGAGCCCTCCTCCAGCCCTTCCTCCA | 37 | 37[a] | 1 | 1 |
| 3 | O-33.15-32 | $(CACCTCTCCACCTGCC)_2$ | 32 | 16 | 2 | 3 |
| 4 | O-33.15-80 | $(CACCTCTCCACCTGCC)_5$ | 80 | 16 | 5 | 2 |
| 5 | O-AY-29 | GAGGARYAGAAAGGYGRGYRVTGTGGGCG [b] | 29 | 37 | 1 | 1 |
| 6 | O-AY-27 | AGGARYAGAAAGGYGRGYRVTGTGGGC | 27 | 37 | 1 | 1 |
| 7 | O-YNH24 | TCCTGAACAACCCCACTGTACTTCCCA | 27 | 31 | 1 | 1 |
| 8 | O-33.1 | GTGCCTGCTTCCCTTCCCTCTCTTGTC | 27 | 62 | 1 | 1 |
| 9 | O-34BHI | CCTGCTCCGCTCACGTGGCCCACGCAC | 27 | Unknown | 1 | 1 |
| 10 | O-CCR-26 | $(CCR)_8CC$ | 26 | 3 | 8.67 | 2 |
| 11 | O-CCA-26 | $(CCA)_8CC$ | 26 | 3 | 8.67 | 2 |
| 12 | O-H-Ras | CACTCCCCCTTCTCTCCAGGGGACGCC | 28 | 28 | 1 | 4 |

a/ The repeat unit for this sequence has been alternatively described as 11 or 37.

b/ R = A and/or G, Y = C and/or T, V = Not T.

As Table I shows, under varying hybridization and wash criteria, these probes fall into four different hybridization pattern categories. Category (1) probes produce highly polymorphic, multiloci, patterns under low hybridization criteria and a locus specific polymorphic pattern under high criteria. Category (2) probes produce a highly polymorphic, multiloci, pattern under both low as well as high criteria, although a few minor bands are lost in subsequent washes. Category (3) prior art probes produce a polymorphic multiloci pattern under low criteria and allele specific but non-polymorphic pattern under high criteria. The category (4) probe produces a locus specific and polymorphic pattern under low as well as high criteria.

Hybridization criteria are based on temperature and cation, typically sodium ion, concentration. Frequently the temperature is varied while the cation concentration is constant.

The following empirical formula has been suggested for the calculation of $T_d$ (the temperature at which one-half of the oligonucleotide duplex becomes dissociated in 1 M NaCl) for a given oligonucleotide:

$$T_d = 4°C (G + C) + 2° (A + T)$$

where (G + C) is the number of G and C bases in the oligonucleotide and (A + T) the number of A and T bases in the oligonucleotide. (See Suggs, et al. "Development Biology Using Purified Genes" (D. Brown and C.F. Fox, Eds.) Academic Press, New York (1981).)

However, finding optimal hybridization and wash conditions is empirical in nature. Hence the formula is but a rule of thumb useful only for most oligonucleotides 11 to 23 bases in length.

For the purposes of this invention for oligonucleotide probes not more than 23 nucleotides in length, high criteria hybridizations may be done at a temperature of from about 0-10°C, preferably about 5°C below $T_d$. For longer oligonucleotide probes, hybridization and washes can be done at 65°C. In each case, post-hybridization washes are accomplished at temperatures sufficiently high to leave only the duplexes where 80% of the nucleotide are base paired.

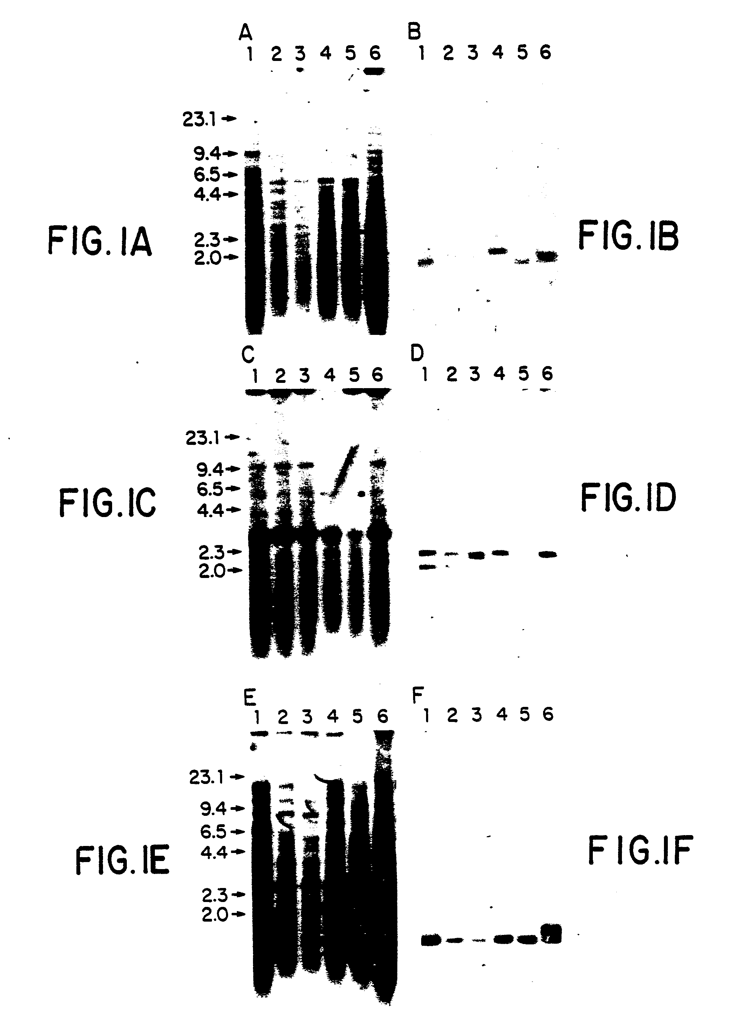

The synthetic oligonucleotide probes listed in Table I were synthesized using an automated DNA synthesizer and purified by polyacrylamide gel electrophoresis and HPLC using a PRP-1 reverse phase column (Hamilton). Oligonucleotide (10 pmole) were labeled at the 5' terminal hydroxyl group using γ-[32P ATP (20 pmole NEN, 7000Ci/mmole) and T4 polynucleotide kinase 5-7 units (NEN) in 10 μl reaction volume containing 67 mM Tris-HCl at pH 8.0, 10 mM Mg (OAc)₂, and 10 mM dithiothreitol. The kinase reactions were carried out at 37°C for 45 min. The labeled oligonucleotides were then separated from the [gamma 32P] ATP by chromatography over DE 52 Cellulose (Whatman) as described elsewhere. (See Ali, et al. (1986) Hum.Genet. 74: 239.) Gels containing samples from unrelated individuals were hybridized to each probe at 37°C. After each hybridization, the gels were washed in 6xSSC at various temperatures ranging from 37°C to 70°C. After each wash, autoradiograms were recorded. Some of the representative hybridization results are presented by Figure 1.

To obtain the data reflected by Figures 1A to 1E, DNA was isolated from blood cells of unrelated individual donors according to the methods of Ali, supra. 3-5 μg DNA was digested with Hinf I (BRL) according to the supplier's specification and electrophoresed in 0.7% agarose gel (Seakem, ME) in recirculating TAE buffer (40 mM Tris, 12 mM sodium acetate, 2 mM EDTA, pH 8.3). For size estimation, phage lambda DNA predigested with Hind III restriction enzyme was included in the gel electrophoresis. After the electrophoresis, the gels were dried on a gel dryer (Bio-Rad) for 1 hr at room temperature followed by another 1 hr at 60°C. Prior to the hybridization, the gels were processed for denaturation and neutralization as described elsewhere. (Jeffreys, et al. (1985) Nature 314:67.) Gel hybridizations were carried out for 16 to 20 hr at 37°C in 5xSSPE [1xSSPE = 180 mM NaCl, 10 mM sodium phosphate, 1 mM EDTA, pH 8.0] 0.1% SDS, 10 μg/ml sonicated and denatured E. coli DNA and 1 x 10⁶ cpm/ml labeled oligonucleotide probe. After the hybridization, gels were washed in 6xSSC at room temperature for 3-4 hr, and exposed to X-ray film (Kodak X-AR) with two intensifying screens (Dupont lightning plus) overnight (Figures 1A, C and E). After obtaining the first autoradiogram, the gels were washed at increasing temperatures including 37, 40, 45, 50, 55, 60, 65°C, and some of the gels at 70°C for 5-10 min or longer depending upon the signal intensity and after each wash, the autoradiograms were obtained (Figures 1B, D and F).

Only the following representative autoradiograms are shown.

Figure 1A: Autoradiograms of the gel hybridized with oligonucleotide probe 0-33.6 (Table I) and exposed without any stringent wash.

Figure 1B: Same gel after a wash at 65°C for 10 min. Note the locus specific polymorphic bands. All the other bands are lost during the sequential washes at the higher temperature (not shown).

Figure 1C: Autoradiogram of the gel hybridized with 0-33.1 probe (Table I). Several polymorphic bands are seen ranging from 10.1 to 1.8kb.

Figure 1D: The same gel after a final wash at 65° C for 10 min. Note the one or two bands in the size range of 2.5 to 1.6kb.

Figure 1E: Autoradiogram of the gel hybridized with the 0.33.15 probe (Table I) and exposed without any stringent wash.

Figure 1F: The same gel after the wash at 65° C for 15 min. All the bands are eliminated except a 1.3 kb band present in all the individuals. In lane 6, there is an additional band of about 1.45kb. This is the only sample that shows this additional band; otherwise, all the individuals are alike.

The first oligonucleotide probe 0-33.6, a 22 base long probe complementary to two consensus repeat units of the Jeffreys 33.6 sequence, (See Ali, supra, note 6.) produces a highly polymorphic pattern giving rise to a number of shared and unshared bands when the gel is exposed without any stringent wash (Fig. 1A). There are four bands of 3.8, 2.8, 2.4 and 2.1 kb which appear to be common in all the individuals. These non-polymorphic fragments appear together with several polymorphic fragments ranging from 6-18 kb. After washing the gel at increasingly higher temperatures, a locus specific hybridization pattern emerges, where all the bands except those in the size range of 1.3 to 1.5 kb are eliminated (Fig. 1B). These bands are obviously polymorphic; however, since the repeat unit of this locus is only 11 nucleotides, the different alleles are not resolved well. These bands which are removed by higher stringency washes may reflect related sequences which are not completely complementary to the probe.

Similarly, the seventh oligonucleotide 0-33.1, a 27 base long probe complementary to a single consensus repeat unit of the 33.1 sequence, produces a complex polymorphic pattern under low stringency conditions (Fig. 1C) comprises mostly of unshared bands in the size range of 9.5 to 1.9 kb, except for one of 10.1 kb which is relatively faint and seems to be common amongst all six individuals. After the high stringency wash, all but one or two bands are removed. Of the six individuals analyzed, four of them have two bands in the size range of 1.7 to 2.5 kb, whereas two of them have only one band each of about 2.5 kb (Fig. 1D). Thus, under high stringency conditions, this oligonucleotide probe revealed five alleles in the six individuals examined (2.5, 2.3, 2.1, 2.0 and 1.7 kb).

In contrast to the results obtained with the two probes discussed above, the second probe 0-33.15, a 32 base long probe complementary to two consensus repeat units of the 33.15 sequence, produced a complex DNA fingerprint pattern when hybridized and washed at low stringency (Fig. 1E) but a non-polymorphic locus specified pattern when washed at high stringency (Fig. 1F). Under the high stringency wash condition, a 1.3 kb non-polymorphic band is seen in all individuals (Fig. 1F).

In addition to this 1.3 kb non-polymeric band, there is an additional 1.45 kb band present in one individual (Fig. 1F, lane 6). It is difficult to account for the presence of this additional band. Only the 1.3 kb band was found when the genomic DNA of several other unrelated individuals was analyzed (not shown). Based on the level of hybridization of the probe to the two bands in this individual, it is reasonable to assume that this additional band is not an allele of the 1.3 kb band but rather is due to the presence of an additional locus complementary to the probe (no family members of this individual are available to confirm this suggestion).

The cloned VTNR sequence 33.6 has been described as having an 11 nucleotide repeat unit or alternatively as having a 37 nucleotide repeat unit made up of three 11 nucleotide repeats plus additional nucleotides. An oligonucleotide 0-33.6-37, a 37 nucleotide long probe 14 complementary to one consensus 37 nucleotide repeat, was synthesized. Genomic DNA from several unrelated individuals was digested with Hinf I and electrophoresed on a 1.5% agarose gel. The DNA in the gel was hybridized with the 37 base long probe under stringent hybridization and post-hybridization wash conditions. As can be seen in Fig. 2A, the 33.6 locus is highly polymorphic, with three of the nine individuals examined being heterozygous. At least 5 alleles are resolved in the 18 chromosomes studies. This probe thus revealed the intrinsic polymorphism of the 33.6 locus which was suggested by the results in Fig. 1A. Genomic DNA from a family was analyzed in the same way (Fig. 2B). The results are consistent with a single Mendelian locus.

The synthetic oligonucleotide probes of this invention are of particular utility for genetic identification purposes, paternity and maternity testing, diagnosis of genetic diseases and cancer, and in the study of post bone marrow transplant chimerism. See, e.g., Yam, et al., Am.J.Hum.Genet. 41:867-861 (1987) and parent application Serial No. 055,224.

## Claims

1. A synthetic oligonucleotide hybridization probe comprising at least one sequence at least 12 nucleotides long, said probe forms duplexes in which at least 80% of the nucleotides are base paired with any nucleotide sequence that is either substantially of the form

(a) $(X_i...X_i)n$ in which X is any nucleotide; i which designates the number of nucleotides in the sequence is at least 2 and n is at least 2, or substantially of the form

(b) $[(X_i...X_i)mY]w$ in which X is any nucleotide, Y is any group of 1 or more nucleotides and is different from $(X_i...X_i)$, m is at least 1, w is 2, and i is at least 2, the number of nucleotides in said probe being less than 3i when i is greater than 3.

2. A synthetic DNA hybridization probe as defined by claim which comprises a mixture of sequences each at least 12 nucleotides long.

3. A synthetic DNA hybridization probe as defined by claim which comprises a single sequence at least 12 nucleotides long.

4. A locus specific synthetic DNA probe which hybridizes at high criteria to form 100% matched duplexes with a nucleotide sequence as described by claim 1.

5. A probe as defined by claim 4 which is not more than 23 nucleotides in length and which forms at least 80% matched duplexes with any sequence as defined in subparagraphs (a) and (b) of claim 1 when hybridized at a temperature of from about 5°C to about 10°C below $T_d$ followed by post-hybridization washes at temperatures sufficiently high to leave only the duplexes where at least 80% of the nucleotides are base paired.

6. A probe as defined by claim 4 which is more than 23 nucleotides in length and which forms at least 80% matched duplexes with any sequences as defined in subparagraphs (a) and (b) of claim 1 when the hybridization is accomplished at 65° and the post-hybridization washes are accomplished at temperatures sufficiently high to leave only duplexes where at least 80% of the nucleotides are base paired.

7. A synthetic oligonucleotide which consists of or comprises any one or more of the sequences:

(a) 5′ (CCTCCAGCCCT) 3′

(b) 5′ (CACCTCTCCACCTGCC) 3′

(c) 5′ GAGGARYAGAAAGGYGRGYRVTGTGGGCG 3′ in which R is A or G, or A and G, Y is C or T, or C and T, and V is not T

(d) 5′ (AGGARYAGAAAGGYGRGYRVTGTGGGC 3′ in which R is A or G

(e) 5′ TCCTGAACAACCCCACTGTACTTCCCA 3′

(f) 5′ GTGCCTGCTTCCCTTCCCTCTCTTGTC 3′

(g) 5′ CCTGCTCCGCTCACGTGGCCCACGCAC 3′

(h) 5′ (CCR)₈CC 3′ in which R is A or G

(i) 5′ (CCR)₈CC 3′

(j) 5′ GCCCTTCCTCCGGAGCCCTCCTC-CAGCCCTTCCTCCA 3′

(k) 5′ AGGARYAGAAAGGYGR-GYRVTGTGGGC 3′

(l) 5′ CACTCCCCCTTCTCTCCAGGG-GACGCC 3′

8. A method of genetically identifying a sample of human genomic DNA which comprises hybridizing said DNA with a synthetic oligonucleotide as defined in claim 1.

9. A method of genetically identifying a sample of human genomic DNA which comprises hybridizing said DNA with a synthetic oligonucleotide as defined in claim 2.

10. A method of genetically identifying a sample of human genomic DNA which comprises hybridizing said DNA with a synthetic oligonucleotide as defined in claim 3.

11. A method of genetically identifying a sample of human genomic DNA which comprises hybridizing said DNA with a synthetic oligonucleotide as defined in claim 4.

12. A method of genetically identifying a sample of human genomic DNA which comprises hybridizing said DNA with a synthetic oligonucleotide as defined in claim 7.

13. A synthetic oligonucleotide probe as defined in claim 1 which, under high criteria yields a locus specific, polymorphic hybridization pattern.

14. A synthetic oligonucleotide as defined by claim 1 which, at high criteria, yields a multiloci polymorphic hybridization pattern.

15. A synthetic oligonucleotide probe as defined in claim 1 which forms said at least 80% matched duplexes at high criteria.

16. A probe as defined in claim 15 not more than 23 nucleotides in length which forms said at least 80% matched duplexes when hybridization is accomplished at a temperature of from about 5°C to about 10°C below $T_d$ and post-hybridization washes are accomplished at temperatures sufficiently high to leave only the duplexes where at least about 80% of the nucleotides are base paired.

FIG. IA

FIG. IB

FIG. IC

FIG. ID

FIG. IE

FIG. IF

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-8 602 948  (LISTER INSTITUTE OF PREVENTIVE MEDICINE)<br>* Whole document * | 1-16 | C 12 Q   1/68 |
| X,P | EP-A-0 238 329  (IMPERIAL CHEMICAL INDUSTRIES PLC)<br>* Page 1, line 3 - page 7, line 57; page 11, example 1; page 15, example 2; page 21, example 3; page 22, line 34 - page 23, line 52; figures 1-12 * | 1-16 | |
| Y | WO-A-8 606 102  (BIOTECHNOLOGY RESEARCH PARTNERS)<br>* Page 4, line 6 - page 5, line 28; page 9, lines 5-32; page 25, table 2; page 30, line 3 - page 33, line 24 * | 1-16 | |
| Y | EP-A-0 164 054  (CETUS CORP.)<br>* Page 4, line 31 - page 6, line 21; page 33, line 2 - page 35, line 28 * | 1-16 | |
| X,D | NATURE, vol. 314, 7th March 1985, pages 67-73, GB; A.J. JEFFREYS et al.: "Hypervariable 'Minisatellite' regions in human DNA"<br>* Page 67, column 2, line 15 - page 71, column 2, line 7; page 72, column 2, lines 7-18 * | 1-16 | TECHNICAL FIELDS SEARCHED (Int. Cl.4)<br><br>C 12 Q   1/00<br>C 12 N   15/00 |
| X | NATURE, vol. 317, 31st October 1985, pages 818-819, GB; A.J. JEFFREYS et al.: "Positive identification of an immigration test-case using human DNA fragments"<br>* Whole article *<br>                    -/- | 1-16 | |

The present search report has been drawn up for all claims

| Place of search | Date of completion of the search | Examiner |
|---|---|---|
| THE HAGUE | 06-09-1988 | VAN BOHEMEN C.G. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P0401)

European Patent
Office

# EUROPEAN SEARCH REPORT

Application Number

EP 88 30 4763

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | NATURE, vol. 316, 4th July 1985, pages 76-79, GB; A.J. JEFFREYS et al.: "Individual-specific "fingerprints" of human DNA" * Whiole article * | 1-16 | |
| X,D | AMERICAN JOURNAL OF HUMAN GENETICS, vol. 39, 1986, pages 11-24, US; A.J. JEFFREYS et al.: "DNA "fingerprints" and segregation analysis of multiple markers in human pedigrees" * Page 11, lines 1-26; page 13, line 11 - page 23, line 15 * | 1-16 | |
| X | JOURNAL OF MEDICAL GENETICS, vol. 23, no. 5, 1986, page 471, US; M.D.A. CRAWFORD et al.: "Minisatellite DNA determination of zygosity in twins discordant for sacral agenesis" * Whole article * | 1-16 | |
| X | CHEMICAL ABSTRACTS, vol. 100, 1984, page 118, abstract no. 80593s, Columbus, Ohio, US; R. YANG et al.: "The nucleotide sequence of a new human repetitive DNA consists of eight tandem repeats of 66 base pairs", & GENE 1983, 25(1), 59-66 * Abstract * | 1 | TECHNICAL FIELDS SEARCHED (Int. Cl.4) |

The present search report has been drawn up for all claims

| Place of search | Date of completion of the search | Examiner |
|---|---|---|
| THE HAGUE | 06-09-1988 | VAN BOHEMEN C.G. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)